# FASCICULE DE BREVET EUROPEEN

(11) **EP 0 498 718 B1**
(45) Date de publication et mention de la délivrance du brevet: **03.08.1994**
(21) Numéro de dépôt: 92400289.2
(22) Date de dépôt: 05.02.1992
(51) Int. Cl.: C07D 211/70, A61K 31/445

(54) **Utilisation de dérivés 4-(3-trifluorométhylphényl)-1,2,3,6-tétrahydropyridiniques comme capteurs de radicaux libres**
Verwendung von 4-(3-trifluormethylphenyl)-1,2,3,6-Tetrahydropyridinderivaten als freie Radikalfänger
Use of 4-(3-trifluoromethylphenyl)-1,2,3,6-tetrahydro-pyridine-derivatives as free radical scavengers

(30) Priorité: 05.02.1991 FR 9101283
(43) Date de publication de la demande: 12.08.1992
(73) Titulaire: ELF SANOFI, 75008 Paris (FR)
(72) Inventeur: Heaulme, Michel, F-34000 Montpellier (FR); Guzzi, Umberto, I-20148 Milano (IT)
(74) Mandataire: Gillard, Marie-Louise

(56) Documents cités:
- EP-A- 0 060 176
- EP-A- 0 101 381
- EP-A- 0 369 887
- EP-A- 0 412 901
- EP-A- 0 458 697
- ANNALS OF THE RHEUMATIC DISEASES, vol. 45, 1986, pages 608-611; T. WOODRUFF etal.: "Is chronic synovitis an example of reperfusion injury?"
- ANNALS OF INTERNAL MEDICINE, vol. 107, no. 4, octobre 1987, pages 526-545,American College of Physicians; C.E. CROSS et al.: "Oxygen radicals and humandisease"

## Description

La présente invention concerne une nouvelle utilisation thérapeutique de certains dérivés tétrahydropyridiniques ainsi que les produits nouveaux ainsi utilisés.

La classe de dérivés tétrahydropyridiniques de formule générale (A) : 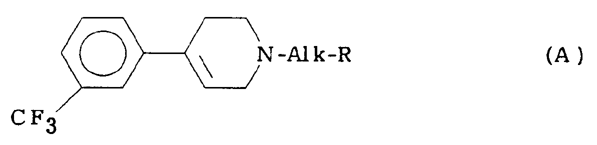
dans laquelle Alk représente un groupe alkylène à chaîne droite ou ramifiée contenant de 2 à 4 atomes de carbone et R est un groupe pyridyle, pyridyle N-oxyde, ou naphtyle non-substitués ou substitués par un groupe alkyle inférieur a été décrite dans la demande de brevet européen EP-A-101 381.

Pour les produits de cette classe une activité anorexigène a été indiquée et revendiquée dans la demande de brevet ci-dessus.

Une demande de brevet ultérieure, EP-A-369,887, décrit l'utilisation de certains dérivés de formule (A) où R est entre autres un groupe naphtyle, en tant qu'agents thérapeutiques pour combattre l'anxiété et les troubles anxio-dépressifs.

On a maintenant trouvé que les composés de formule (A) ci-dessus, dans laquelle R est un groupe 2-naphtyle et Alk représente un groupe éthylène, éventuellement substitué par un ou deux groupes méthyle sur l'atome de carbone lié au groupe R, ainsi que leurs sels d'addition, ont une activité très intéressante en tant que capteurs ("scavengers") de radicaux libres.

Un premier objet spécifique de la présente invention est donc l'utilisation d'au moins un composé de formule (I) 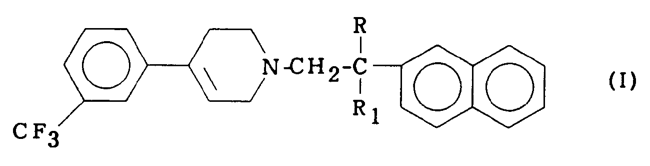
dans laquelle R et R₁, chacun indépendamment, peuvent représenter un atome d'hydrogène ou un groupe méthyle, ou l'un de ses sels d'addition d'acides pharmaceutiquement acceptables, pour la préparation de compositions pharmaceutiques pour le traitement ou la prophylaxie de toutes pathologies dans lesquelles les radicaux libres sont impliqués.

Les radicaux libres sont des espèces chimiques contenant un ou plusieurs électrons non appariés qui, à cause de leur haute réactivité, peuvent amorcer des réactions en chaîne, non controlables, au niveau cellulaire.

Ils peuvent ainsi endommager, de façon réversible ou irréversible, des composés de toute classe biochimique, y compris les protéines, les amino-acides, les lipides, les lipoprotéines, les acides nucléiques, et les carbohydrates en altérant donc les activités cellulaires primordiales telles que le fonctionnement membranaire, le métabolisme et l'expression des gènes.

Il y a beaucoup de pathologies dans lesquelles un rôle important des radicaux libres, soit comme agents étiologiques primaires ou secondaires, soit comme amplificateurs des maladies originales, a été envisagé (cfr. Oxygen Radicals and Human Disease - *Annals of Internal Medecine*, *1987*, *107*, *526*-*545* et plus particulièrement le Tableau 1 à la page 527 qui est inclus dans le texte de la présente demande à titre de référence).

Des preuves substantielles ont été acquises à l'appui du rôle des radicaux libres dans la dégénérescence cérébrale et de la moelle épinière post-traumatisme et dans les lésions tissulaires provoquées par l'ischémie-reperfusion (B. Halliwell ed., *Proceedings of the Upjohn Symposium on Oxidants and Disease-Bethesda:* *Federation of American Societies for Experimental Biology*, *1987*, et J. M McCord, *N. Eng. J. Med., 1985,* *312**, 159-163*).

Il semble certain en effet que dans les ischémies, les lésions secondaires à l'hypoxie soient moins graves que les lésions provoquées par le flux élevé, dans les tissus ischémiques, des radicaux libres dont la formation est déclenchée par l'oxygène moléculaire, dès que la circulation du sang est rétablie.

Il a été aussi démontré que les lésions qui se produisent au cours de la reperfusion ne se limitent pas au niveau cardiaque et/ou cérébral mais peuvent aussi atteindre le rein, le foie, le pancréas et le système endothélial vasculaire et musculaire lisse.

Particulièrement évident est le rôle des radicaux oxygène dans les lésions myocardiques provoquées par l'ischémie-reperfusion (S.R. Jolly et al., *Circ. Res., 1984,* *54**, 277-85*; L.C. Becker et al., *Prog. Cardiovasc. Dis., 1987,* *30**, 23-44;* et R. Bolli et al., *Circ. Res. 1989,* *65,* *607-622).*

On a démontré que certaines lésions provoquées par les radicaux libres, peuvent aussi se manifester dans les articulations rheumatoides enflammées (T. Woodruff et al., *Am. Rheum. Dis.,* *45**, 608-611, 1986).*

En général, les composés de formule (I) peuvent être utilisés en thérapie dans tous les processus pathologiques qui entraînent des dommages cellulaires car il a été démontré que dans tous ces processus, des radicaux oxygène sont impliqués.

Plus précisement, des applications cliniques pour lesquelles on peut bien envisager l'utilisation des composés de formule (I) comprennent donc la protection contre la dégénérescence cérébrale et de la moelle épinière post-traumatisme et contre les lésions dues à l'ischémie-reperfusion.

D'autres applications cliniques dans lesquelles les composés de formule (I) et leurs sels d'addition d'acides pharmaceutiquement acceptables, en tant que capteurs de radicaux libres, peuvent être utilisés, comprennent la protection contre les lésions dans la cornée ou dans la rétine dues aux radicaux oxygène, telles que la cataracte d'origine oxydative et la rétinopathie du prématuré; la protection des organes, notamment du rein, dans les transplantations; la protection contre les lésions provoquées par les irradiations; la prévention de rechutes dans le cas d'ulcères duodénaux; et la protection contre les lésions tissulaires provoquées par certains agents toxiques dont la toxicité est due à la formation de radicaux libres.

A ces fins, les composés de formule (I) ainsi que leurs sels pharmaceutiquement acceptables peuvent être convenablement administrés par voie orale, parentérale, sublinguale, transdermique ou topique, élaborés sous forme de compositions pharmaceutiques.

Ces compositions pharmaceutiques renferment au moins un produit choisi parmi les composés de formule (I) et leurs sels d'addition pharmaceutiquement acceptables, en association avec un véhicule pharmaceutiquement inerte.

Acides tant organiques qu'inorganiques peuvent être utilisés pour former des sels d'addition d'acides des composés de formule (I) non toxiques et pharmaceutiquement acceptables.

On cite par exemple les acides sulfurique, nitrique, phosphorique, chlorhydrique, citrique, acétique, lactique, tartrique, pamoïque, éthanedisulfonique, sulfonique, succinique, cyclohexylsulfonique, fumarique, maléique et benzoïque.

En ce qui concerne l'administration par voie orale ou sublinguale, on utilise en particulier des comprimés, simples ou dragéifiés, des gélules, des granules éventuellement à libération retardée, des gouttes ou encore des liposomes. En ce qui concerne l'administration par voie intraveineuse, sous-cutanée ou intramusculaire, on a recours à des solutions stériles ou stérilisables en particulier pour perfusion veineuse, tandis que l'on peut réaliser des patches conventionnels pour l'administration par voie transdermique. Pour l'utilisation topique on peut utiliser des crèmes ou des lotions à étendre sur la peau, des solutions ou des pommades pour les yeux.

Les compositions pharmaceutiques selon la présente invention peuvent être préparées selon des méthodes usuelles bien connues dans le domaine de la technique pharmaceutique.

Le principe actif peut être incorporé dans des excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Les compositions pharmaceutiques de l'invention peuvent avantageusement contenir un composé de formule (I) ou l'un de ses sels d'addition pharmaceutiquement acceptables en association avec un ou plusieurs autres médicaments connus et communément utilisés pour les mêmes indications thérapeutiques.

La quantité de principe actif à administrer par jour, selon la méthode de la présente invention, dépend de la particularité de l'indication thérapeutique, de la gravité des affections à traiter, ainsi que du poids du malade et de la voie d'administration.

Pour une administration systémique, la dose globale chez l'homme varie généralement entre 2 et 900 mg par jour, par exemple de 3 à 500 mg, et plus convenablement de 10 à 300 mg par jour.

Des formes unitaires de dosage pour l'administration systémique comprendront généralement de 2 à 300 mg, allant de préférence de 5 à 150 mg, y compris par exemple le dosage de 5 à 50 mg (à savoir 5, 10, 20, 30, 40, et 50 mg de produit). Ces doses unitaires seront administrées normalement une ou plusieurs fois par jour, de préférence une à trois fois par jour.

Pour l'administration topique, les compositions pharmaceutiques contiennent généralement de 0,0001 à 1% de principe actif et de préférence de 0,01 à 0,5%. La forme unitaire de dosage pour l'administration topique sur l'oeil (goutte) comprendra généralement de 10 ng à 10 mg et de préférence de 100 ng à 1 mg.

Un autre objet spécifique de la présente invention est donc une méthode pour la prophylaxie et le contrôle de toutes pathologies impliquant des radicaux libres, qui comprenne l'administration, aux mammifères qui en ont besoin, d'une dose efficace d'au moins un composé de formule (I) ou d'un de ses sels pharmaceutiquement acceptables.

Les composés de formule (I) dans laquelle l'un des radicaux R et R₁, est un groupe méthyle et l'autre est un atome d'hydrogène ou un groupe méthyle ainsi que leurs sels d'addition d'acides, sont des produits nouveaux et réprésentent un autre objet spécifique de la présente invention.

Le composé de formule (I) où l'un de R et R₁ est un groupe méthyle et l'autre est un atome d'hydrogène possède un centre chiral.

La présente invention concerne les isomères purs de ce composé ainsi que tout mélange de ceux-ci.

Ces composés peuvent être préparés, selon une méthode générale, par réduction de l'amide correspondant de formule (II) 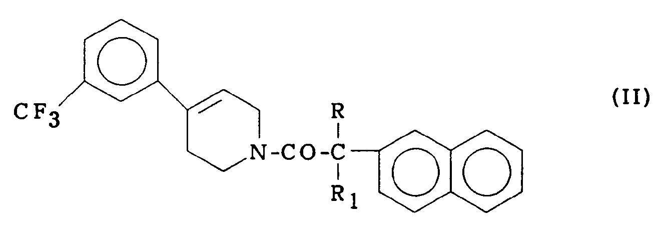
dans laquelle un des radicaux R et R₁ est un groupe méthyle et l'autre est un atome d'hydrogène ou un groupe méthyle.

Cette réduction peut être convenablement réalisée par un hydrure d'aluminium ou par un hydrure complexe de lithium et d'aluminium dans un solvant organique inerte à une température entre 0°C et la température de reflux du mélange réactionnel. Le produit ainsi obtenu peut être éventuellement transformé en l'un de ses sels d'addition d'acides.

La réaction de réduction est conduite selon les modes opérationnels connus en soi en utilisant comme agent de réduction l'hydrure d'aluminium ou un hydrure complexe de lithium et d'aluminium, tel que LiALH₄, LiAlH(OCH₃)₃ et similaires. On opère en général dans un solvant inerte comme un éther, tel que l'éther diéthylique, le tétrahydrofurarine, le dioxane ou le 1,2-diméthoxyéthane.

Selon un mode opérationnel préférentiel, on opère avec une quantité en moles d'hydrure de lithium et d'aluminium, LiAlH₄, double par rapport au composé (II) de départ, à une température de 20-30°C dans de l'éther diéthylique et sous atmosphère inerte. Après environ 1 h, la réduction est complète et le composé voulu est isolé selon les techniques conventionnelles sous forme de base libre ou d'un de ses sels.

La base libre peut être transformée en l'un de ses sels par simple salification dans un solvant organique tel qu'un alcool, de préférence éthanol ou isopropanol, un éther comme le 1,2-diméthoxyéthane, l'acétate d'éthyle ou un hydrocarbure comme l'hexane.

Les composés de formule (II) ci-dessus sont préparés en faisant rèagir la 4-(3-trifluorométhylphényl)-1,2,3,6-tétrahydropyridine de formule (III) 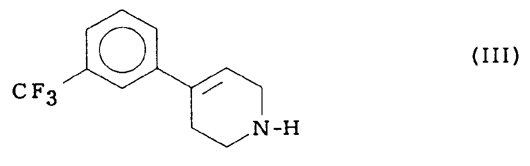
avec un dérivé fonctionnel d'un acide carboxylique de formule (IV) 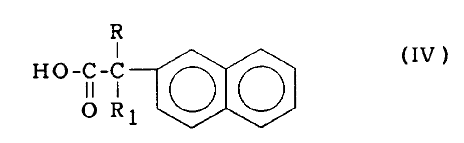
où un des radicaux R et R₁ est un groupe méthyle et l'autre est un atome d'hydrogène ou un groupe méthyle, dans un solvant organique à une température comprise entre -10°C et la température de reflux du mélange réactionnel.

Comme dérivé fonctionnel approprié, on peut utiliser l'acide libre activé (par exemple avec le BOP), l'anhydride, un anhydride mixte, un ester actif ou un halogénure d'acide, le chlorure de préférence. Parmi les esters actifs, l'ester de p-nitrophényle est particulièrement préféré, mais les esters de méthoxyphényle, de trityle, de benzhydryle et similaires sont également convenables.

La température de réaction peut varier entre -10°C et la température de reflux, mais en général on opère à la température ambiante ou à 30-50°C. Il peut être préférable de réaliser la réaction au froid lorsqu'elle est exothermique, comme dans le cas où on utilise le chlorure en tant que dérivé fonctionnel de l'acide de formule (IV).

Comme solvant de réaction, on utilise de préférence un alcool, tel que le méthanol ou l'éthanol, ou un solvant halogéné tel que le chlorure de méthylène, le dichloroéthane, le chloroforme et similaires, mais d'autres solvants organiques compatibles avec les réactifs employés, par exemple le dioxane, le tétrahydrofuranne ou un hydrocarbure tel que l'hexane, peuvent être également employés.

La réaction peut être convenablement conduite en présence d'un accepteur de protons, par exemple d'un carbonate alcalin ou d'une amine tertiaire.

Le produit que l'on obtient à la fin de la réaction est en général une huile qui peut être isolée et caracterisée selon les techniques conventionnelles, mais qui peut être utilisée à l'état brut pour la réduction avec l'hydrure.

En variante les nouveaux composés de formule (I) peuvent également être préparés par réaction de la 4-(3-trifluorométhylphényl)-1,2,3,6-tétrahydro-pyridine (III) avec un composé de formule (V) 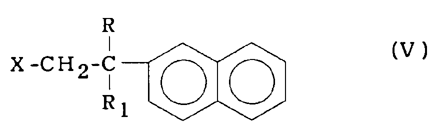
dans laquelle un des radicaux R et R₁ est un groupe méthyle et l'autre est un atome d'hydrogène ou un groupe méthyle et X réprésente un atome de chlore, brome, ou d'iode ou un groupe attracteur d'électrons tel que le groupe méthanesulfonyloxy ou p-toluènesulfonyloxy.

La réaction est conduite dans un solvant organique et à une température comprise entre la température ambiante et 200°C.

Comme solvant organique préférentiel, on utilise un alcool aliphatique ayant de 1 à 6 atomes de carbone, tel que le méthanol, éthanol, n-butanol, n-pentanol, mais d'autres solvants tels que hexane, diméthylformamide, diméthylsulfoxyde, sulfolane, acétonitrile, pyridine et similaires peuvent être utilisés.

La réaction est avantageusement conduite en présence d'un agent de condensation basique, tel que la triéthylamine, surtout dans le cas où X est un atome d'halogène.

La température de réaction peut varier entre la température ambiante (environ 20°C) et 200°C et les temps de réaction varient en conséquence. En général, après 4 à 5 heures de chauffage à 100-150°C, la réaction est complète et le produit final ainsi obtenu peut être isolé selon les techniques conventionnelles et éventuellement transformé en l'un de ses sels par simple salification dans un solvant organique tel qu'un alcool, de préférence éthanol ou isopropanol, un éther comme le 1,2-diméthoxyéthane, l'acétate d'éthyle ou un hydrocarbure comme l'hexane.

Encore, quand on veut obtenir un composé de formule (I) dans laquelle l'un des radicaux R et R₁ est un groupe méthyle et l'autre est un atome d'hydrogène on peut également faire réagir la 4-(3-trifluorométhylphényl)-1,2,3,6-tétrahydropyridine (III) avec le composé (VI) 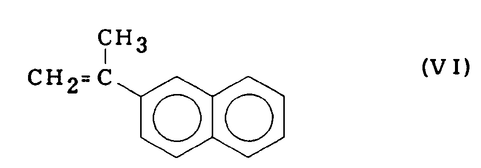
en présence d'un agent de condensation basique.

### PREPARATION I

**Chlorhydrate de 1-[4-(3-trifluorométhylphényl)-1,2,3,6-tétrahydropyrid-1-yl]-2-(napht-2-yl)propane**
**a)** On chauffe à 40°C pendant 2 heures une solution de l'ester éthylique de l'acide 2-(napht-2-yl)propanoique (8 g, 0,035 moles) et de l'hydroxyde de potassium (8 g) dans un mélange de méthanol (80 ml) et d'eau (80 ml). On concentre à sec, on dissout le résidu dans l'eau (100 ml) et on y ajoute de l'acide chlorhydrique concentré jusqu'à pH 3,5. On récupère le précipité par filtration, on le dissout dans de l'éther éthylique et on filtre la solution ainsi obtenue sur celite.
   On concentre à sec en obtenant 4,4 g d'acide 2-(napht-2-yl)-propanoïque. P.f. 112- 114°C
**b)** On agite pendant 1 heure à température ambiante une solution du produit obtenu à l'étape a) ci-dessus (4,2 g, 0,021 moles) et d'1,1-carbonyldiimidazole (3,5 g, 0,021 moles) dans du tétrahydrofuranne (40 ml). On y ajoute une solution de 4-(3-trifluorométhylphényl)-1,2,3,6-tétrahydropyridine (4 g, 0,018 moles) dans du tétrahydrofuranne (20 ml), on agite pendant 2 heures à température ambiante et on concentre à sec. On dissout le résidu ainsi obtenu dans de l'éther éthylique, on lave successivement à l'eau, avec une solution 2N d'acide chlorhydrique, à l'eau, avec une solution de NaHCO₃ à 10% et enfin à l'eau. On filtre sur celite et on concentre à sec en obtenant 6,3 g d'un produit huileux constitué par l′1-[4-(3-trifluorométhylphényl)-1,2,3,6-tétrahydropyrid-1-yl]-1-oxo-2-(napht-2-yl)propane.
**c)** On ajoute goutte à goutte une solution du composé obtenu à l'étape b) ci-dessus (6,3 g, 0,015 moles) dans de l'éther éthylique (60 ml) dans une suspension de LiAlH₄ (1,15 g, 0,031 moles) dans de l'éther éthylique (20 ml) et on agite le mélange réactionnel à température ambiante pendant 2 heures.
   On détruit l'agent réducteur en excès en y ajoutant très lentement de l'eau, on filtre et on concentre le filtrat à sec. On dissout le résidu dans de l'acétone et on précipite le composé indiqué dans le titre, en tant que produit brut, par addition d'acide chlorhydrique gazeux. On sépare le précipité par filtration, on obtient la base libre par neutralisation avec de l'hydroxyde de sodium, extraction à l'acétate d'éthyle et évaporation du solvant organique. On purifie le produit sous forme de base libre ainsi obtenu par chromatographie flash en utilisant en tant qu'éluant un mélange cyclohexane/acétate d'éthyle 9/1, et on le transforme en chlorhydrate correspondant par traitement avec de l'acide chlorhydrique gazeux dans du propanol. On laisse cristalliser et on filtre en obtenant 1,5 g du produit indiqué dans le titre. P.f. 178-180°C.

### PREPARATION II

**Chlorhydrate de 1-[4-(3-trifluorométhylphényl)-1,2,3,6-tétrahydropyrid-1-yl]-2-méthyl-2-(napht-2-yl)propane**
**a)** On chauffe au reflux pendant 2 heures une solution de l'ester éthylique de l'acide 2-méthyl-2-(napht-2-yl)-propanoique (7 g, 0,028 moles), et de l'hydroxyde de potassium (7 g) dans un mélange d'eau (40 ml) et d'éthanol (40 ml). On concentre à sec, on dissout le résidu dans de l'eau et on lave la solution aqueuse à l'éther éthylique. On y ajoute de l'acide chlorhydrique concentré jusqu'à pH 1, on recupère le précipité par filtration et on le cristallise dans de l'éthanol 50°(40 ml) en obtenant 3 g d'acide 2-méthyl-2-(napht-2-yl)propanoïque. P.f. 125-127°C.
**b)** On laisse à température ambiante pendant 4 h une solution du composé obtenu à l'étape a) ci-dessus (3 g, 0,014 moles), de 4-(3-trifluorométhylphényl)-1,2,3,6-tétrahydropyridine (3,6 g, 0,014 moles), de benzotriazol-1yloxytris-(diméthylamino)-phosphonium hexafluorophosphate (BOP) (5,6 g, 0,014 moles) et de triéthylamine (4 ml, 0,028 moles) dans du chlorure de méthylène (40 ml). On concentre à sec, on réprend le résidu dans de l'acétate d'éthyle, on lave successivement à l'eau, avec une solution 2N d'acide chlorhydrique, à l'eau, avec une solution 2N d'hydroxyde de sodium et à l'eau. On filtre sur celite et on concentre en obtenant 4 g de 1-[4-(3-trifluorométhylphényl)-1,2,3,6-tétrahydropyrid-1-yl]-1-oxo-2-méthyl-2(napht-2-yl)propane.
**c)** On ajoute goutte à goutte une solution du composé obtenu à l'étape b) ci-dessus (4 g, 0,0094 moles) dans de l'éther éthylique (40 ml) dans une suspension de LiAlH₄ (0,7 g, 0,0184 moles) dans de l'éther éthylique (40 ml). On agite à température ambiante pendant 4 heures et on détruit l'agent réducteur en excès en y ajoutant très lentement de l'eau (1,5 g). On filtre, on lave la solution à l'eau et on concentre à sec. On purifie le residu ainsi obtenu par chromatographie flash en éluant avec un mélange cyclohexane/acétate d'éthyle 15/1. On obtient 1,4 g de base libre qu'on dissout dans de l'isopropanol. On y ajoute de l'acide chlorhydrique dans de l'éthanol et on laisse cristalliser en obtenant 1,2 g du produit indiqué dans le titre. P.f. 231-33°C.

### EVALUATION PHARMACOLOGIQUE

Les proprietés antiradicalaires des composés de formule (I), ont été évaluées sur un test de péroxydation lipidique conduit de la façon suivante:
des homogénats de cerveau de rat (1,5 mg/ml) ont été mis en incubation dans 100 µl de tampon de Krebs (constitué par 15 mM d'HEPES, pH 7,4, 10 mM de glucose, 140 mM de NaCl, 3,6 mM de KCl, 1,5 mM de CaCl₂, 1,4 mM de KH₂PO₄, et 0,7 mM de MgCl₂), à 37°C, pendant 20 minutes à la suite de l'addition de 200 µM de Fe²⁺, utilisés pour amorcer les réactions.
La mesure de la peroxydation lipidique a été appréciée selon la méthode décrite par J. Braughler et al., *J. Biol.Chem., 261, 10282-10289 (1986)*, en évaluant la quantité de produits TBAR (thiobarbituric acid-reactive oxidation products) qui se forment dans les 20 minutes d'incubation.
En ajoutant des concentrations variables des composés de formule (I) dans le tampon de Krebs, on a pu aisément calculer les CI₅₀ de ces composés, c'est-à-dire les concentrations des composés qui provoquent une inhibition de 50% de la peroxydation lipidique obtenue dans les témoins.
Dans ce test les composés de formule (I) se sont montrés très actifs. En particulier, le composé de formule (I) dans laquelle R et R₁ sont deux atomes d'hydrogène, sous forme de chlorhydrate, est caracterisé par une CI₅₀ de l'ordre de 1-2 µM.
Les composés des Préparations I et II, qui ont montré plus ou moins le même niveau d'activité, ont l'avantage, par rapport au composé de formule (I) où R et R₁ sont l'hydrogène, d'avoir une activité sérotoninergique très limitée (le composé de la Préparation I) ou tout à fait nulle (le composé de la Préparation II).

## Revendications (Revendications pour l'(les) Etat(s) contractant(s) suivant(s): AT, BE, CH, DE, DK, FR, GB, IT, LI, LU, NL, PT, SE)

1. Utilisation d'au moins un composé de formule (I) 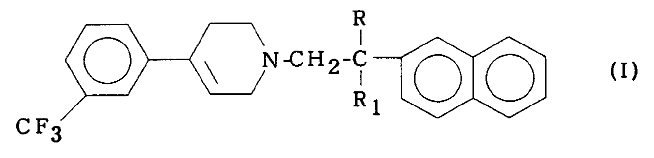 dans laquelle R et R₁, chacun indépendamment, représentent un atome d'hydrogène ou un groupe méthyle, ou un de ses sels d'addition d'acides pharmaceutiquement acceptables pour la préparation d'un médicament pour le traitement et/ou la prophylaxie de toutes pathologies impliquant des radicaux libres.

2. Utilisation selon la revendication 1 pour la préparation d'un médicament pour le traitement et/ou la prophylaxie des processus pathologiques qui entraînent des dommages cellulaires.

3. Utilisation selon la revendication 2 pour la préparation d'un médicament pour le contrôle et la prophylaxie de la dégénérescence cérébrale et de la moelle épinière post-traumatisme, des lésions dues à l'ischémie-reperfusion, des lésions dans la cornée ou la rétine dues aux radicaux oxygène, des lésions des organes lors des transplantations, des lésions provoquées par les irradiations, des rechutes dans le cas des ulcères duodénaux, des lésions tissulaires provoquées par les agents toxiques qui forment des radicaux libres et des lésions provoquées par les radicaux libres dans les articulations rhéumatoides enflammées.

4. Utilisation selon l'une quelconque des revendications de 1 à 3, dans laquelle le composé de formule (I) est le 1-[4-(3-trifluorométhylphényl)-1,2,3,6-tétrahydropyrid-1-yl]-2-(napht-2-yl)-éthane ou l'un de ses sels d'addition d'acides pharmaceutiquement acceptables.

5. Composé de formule (I) 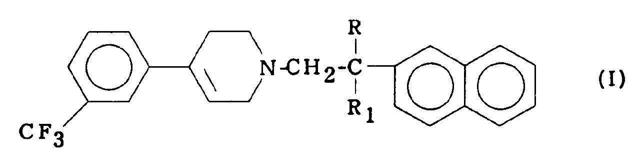 dans laquelle l'un des radicaux R et R₁ est un groupe méthyle et l'autre est un atome d'hydrogène ou un groupe méthyle, et ses sels d'addition d'acides.

6. Composé selon la revendication 5 choisi parmi le 1-[4-(3-trifluorométhylphényl)1,2,3,6-tétrahydropyrid-1-yl]-2-(napht-2-yl)propane et ses sels d'addition d'acides.

7. Composé selon la revendication 5 choisi parmi le 1-[4-(3-trifluorométhylphényl)-1,2,3,6-tétrahydropyrid-1-yl]-2-méthyl-2-(napht-2-yl)propane et ses sels d'addition d'acides.

8. Procédé pour la préparation d'un composé de formule (I) 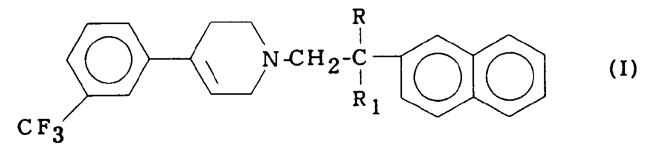 dans laquelle l'un des radicaux R et R₁ est un groupe méthyle et l'autre est un atome d'hydrogène ou un groupe méthyle, et de ses sels d'addition d'acides qui comprend :
a) la réduction de l'amide correspondant de formule (II) 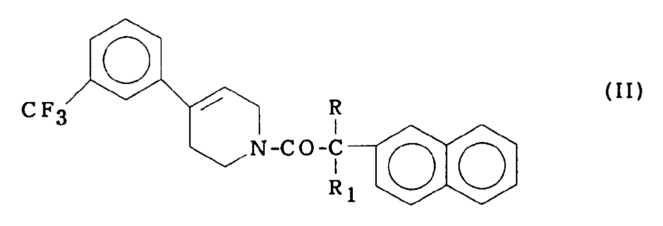 dans laquelle R et R₁ sont tels que définis ci-dessus; ou
b) la réaction entre la 4-(3-trifluorométhylphényl)-1,2,3,6-tétrahydropyridine de formule (III) 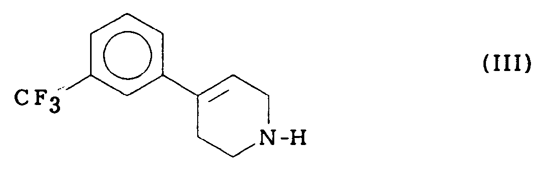 et un composé de formule (V) 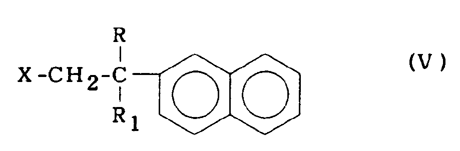 dans laquelle R et R₁ sont tels que définis ci-dessus et X réprésente un atome de chlore, brome, ou iode ou un groupe attracteur d'électrons ;
ou
c) quand on veut obtenir un composé de formule (I) dans laquelle l'un des radicaux R et R₁ est un groupe méthyle et l'autre est un atome d'hydrogène, la réaction entre le composé de formule (III) et le composé (VI) 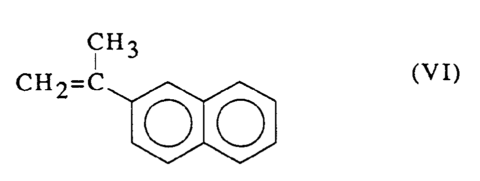 en présence d'un agent de condensation basique;
éventuellement suivie par la conversion du produit ainsi obtenu par l'un de ses sels d'addition d'acides.

9. Composition pharmaceutique renfermant en tant que principe actif au moins un composé de formule (I) 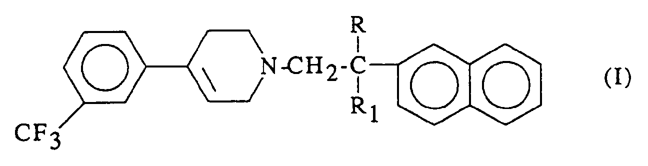 dans laquelle l'un des radicaux R et R₁ est un groupe méthyle et l'autre est un atome d'hydrogène ou un groupe méthyle ou un de ses sels d'addition d'acides pharmaceutiquement acceptables.

10. Composé de formule (II) 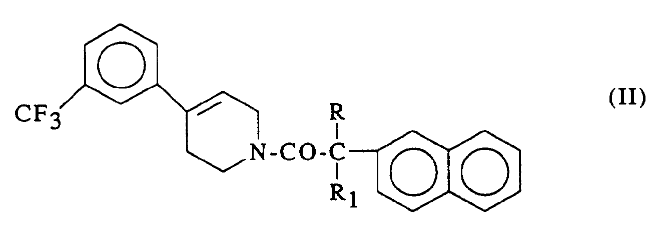 dans laquelle l'un des radicaux R et R₁ est un groupe méthyle et l'autre est un atome d'hydrogène ou un groupe méthyle.

## Revendications (Revendications pour l'(les) Etat(s) contractant(s) suivant(s): ES)

1. Utilisation d'au moins un composé de formule (I) 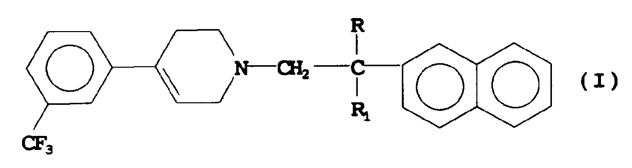 dans laquelle R et R₁, chacun indépendamment, représentent un atome d'hydrogène ou un groupe méthyle, ou un de ses sels d'addition d'acides pharmaceutiquement acceptables pour la préparation d'un médicament pour le traitement et/ou la prophylaxie de toutes pathologies impliquant des radicaux libres.

2. Utilisation selon la revendication 1 pour la préparation d'un médicament pour le traitement et/ou la prophylaxie des processus pathologiques qui entraînent des dommages cellulaires.

3. Utilisation selon la revendication 2 pour la préparation d'un médicament pour le contrôle et la prophylaxie de la dégénérescence cérébrale et de la moelle épinière post-traumatisme, des lésions dues à l'ischémie-reperfusion, des lésions dans la cornée ou la rétine dues aux radicaux oxygène, des lésions des organes lors des transplantations, des lésions provoquées par les irradiations, des rechutes dans le cas des ulcères duodénaux, des lésions tissulaires provoquées par les agents toxiques qui forment des radicaux libres et des lésions provoquées par les radicaux libres dans les articulations rhéumatoides enflammées.

4. Utilisation selon l'une quelconque des revendications de 1 à 3, dans laquelle le composé de formule (I) est le 1-[4-(3-trifluorométhylphényl)-1,2,3,6-tétrahydropyrid-1-yl]-2-(napht-2-yl)-éthane ou l'un de ses sels d'addition d'acides pharmaceutiquement acceptables.

5. Procédé pour la préparation d'un composé de formule (I) 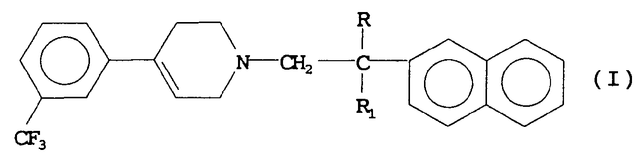 dans laquelle l'un des radicaux R et R₁ est un groupe méthyle et l'autre est un atome d'hydrogène ou un groupe méthyle, et de ses sels d'addition d'acides qui comprend :
a) la réduction de l'amide correspondant de formule (II) 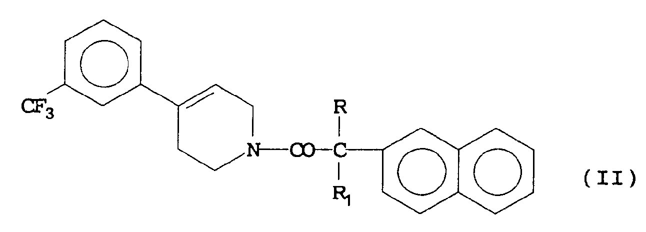 dans laquelle R et R₁ sont tels que définis ci-dessus; ou
b) la réaction entre la 4-(3-trifluorométhylphényl)-1,2,3,6-tétrahydropyridine de formule (III) 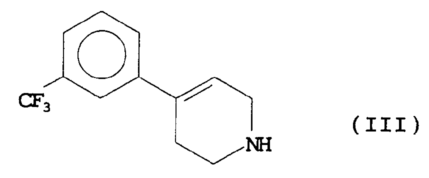 et un composé de formule (V) 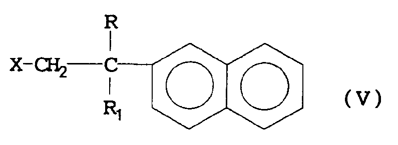 dans laquelle R et R₁ sont tels que définis ci-dessus et X représente un atome de chlore, brome, ou iode ou un groupe attracteur d'électrons ;
ou
c) quand on veut obtenir un composé de formule (I) dans laquelle l'un des radicaux R et R₁ est un groupe méthyle et l'autre est un atome d'hydrogène, la réaction entre le composé de formule (III) et le composé (VI) 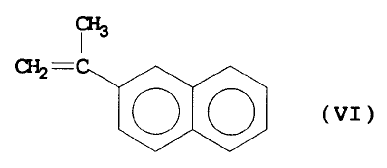 en présence d'un agent de condensation basique ;
éventuellement suivie par la conversion du produit ainsi obtenu par l'un de ses sels d'addition d'acides.

6. Procédé selon la revendication 5, pour la préparation du 1-[4-(3-triflurométhylphényl)-1,2,3,6-tétrahydropyrid-1-yl]-2-(napht-2-yl)propane ou d'un de ses sels d'addition d'acides, qui consiste à réduire l'amide correspondant de formule : 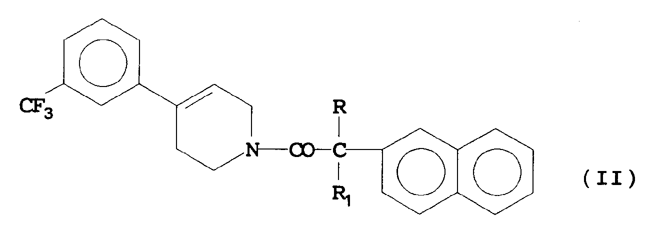 dans laquelle R est un atome d'hydrogène et R₁ est un groupe méthyle,
et à convertir éventuellement le produit ainsi obtenu en l'un de ses sels d'addition d'acides.

7. Procédé selon la revendication 5, pour la préparation du 1-[4-(3-trifluorométhylphényl)-1,2,3,6-tétrahydropyrid-1-yl]-2-méthyl-2-(napht-2-yl]propane ou d'un de ses sels d'addition d'acides, qui consiste à réduire l'amide correspondant de formule : 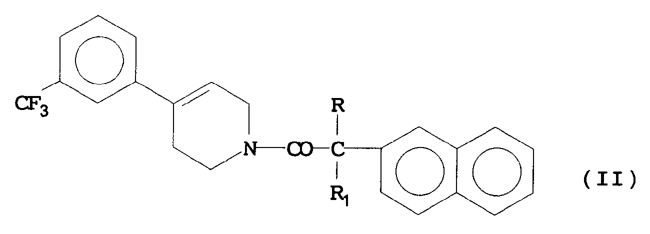 dans laquelle R et R₁ sont chacun un groupe méthyle,
et à convertir éventuellement le produit ainsi obtenu en l'un de ses sels d'addition d'acides.

8. Procédé pour la préparation d'une composition pharmaceutique renfermant en tant que principe actif au moins un composé de formule (I) 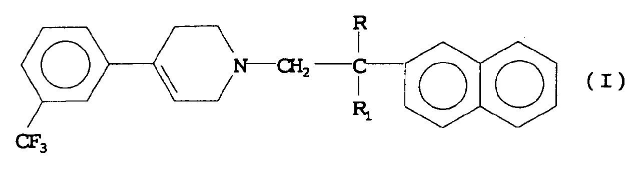 dans laquelle l'un des radicaux R et R₁ est un groupe méthyle et l'autre est un atome d'hydrogène ou un groupe méthyle ou un de ses sels d'addition d'acides pharmaceutiquement acceptables, qui consiste à mélanger ledit principe actif avec un excipient pharmaceutiquement acceptable.

9. Procédé pour la préparation d'un composé de formule (II) 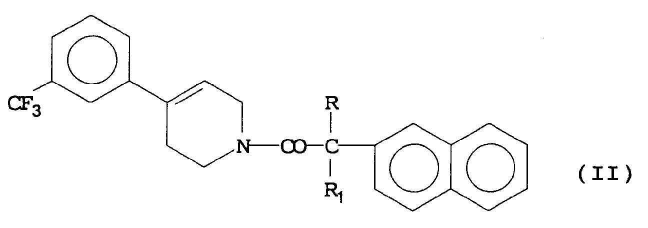 dans laquelle l'un des radicaux R et R₁ est un groupe méthyle et l'autre est un atome d'hydrogène ou un groupe méthyle,
qui consiste à faire réagir la 4-(3-trifluorométhylphényl)-1,2,3,6-tétrahydropyridine de formule (III) 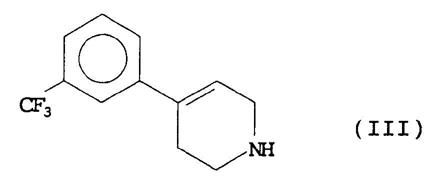 avec un dérivé fonctionnel de l'acide carboxylique de formule (IV) 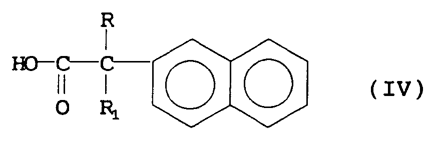 dans laquelle R et R₁ sont tels que définis ci-dessus, dans un solvant organique à une température comprise entre -10° C et la température de reflux du mélange réactionnel.

## Revendications (Revendications pour l'(les) Etat(s) contractant(s) suivant(s): GR)

1. Utilisation d'au moins un composé de formule (I) 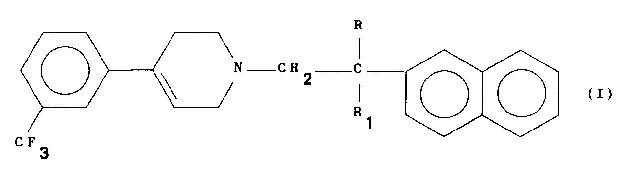 dans laquelle R et R₁, chacun indépendamment, représentent un atome d'hydrogène ou un groupe méthyle, ou un de ses sels d'addition d'acides pharmaceutiquement acceptables pour la préparation d'un médicament pour le traitement et/ou la prophylaxie de toutes pathologies impliquant des radicaux libres.

2. Utilisation selon la revendication 1 pour la préparation d'un médicament pour le traitement et/ou la prophylaxie des processus pathologiques qui entraînent des dommages cellulaires.

3. Utilisation selon la revendication 2 pour la préparation d'un médicament pour le contrôle et la prophylaxie de la dégénérescence cérébrale et de la moelle épinière post-traumatisme, des lésions dues à l'ischémie-reperfusion, des lésions dans la cornée ou la rétine dues aux radicaux oxygène, des lésions des organes lors des transplantations, des lésions provoquées par les irradiations, des rechutes dans le cas des ulcères duodénaux, des lésions tissulaires provoquées par les agents toxiques qui forment des radicaux libres et des lésions provoquées par les radicaux libres dans les articulations rhéumatoides enflammées.

4. Utilisation selon l'une quelconque des revendications de 1 à 3, dans laquelle le composé de formule (I) est le 1-[4-(3-trifluorométhylphényl)-1,2,3,6-tétrahydropyrid-1-yl]-2-(napht-2-yl)-éthane ou l'un de ses sels d'addition d'acides pharmaceutiquement acceptables.

5. Composé de formule (I) 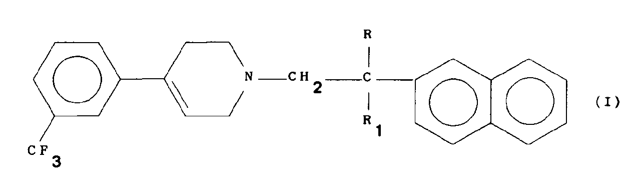 dans laquelle l'un des radicaux R et R₁ est un groupe méthyle et l'autre est un atome d'hydrogène ou un groupe méthyle, et ses sels d'addition d'acides.

6. Composé selon la revendication 5 choisi parmi le 1-[4-(3-trifluorométhylphényl)1,2,3,6-tétrahydropyrid-1-yl]-2-(napht-2-yl)propane et ses sels d'addition d'acides.

7. Composé selon la revendication 5 choisi parmi le 1-[4-(3-trifluorométhylphényl)-1,2,3,6-tétrahydropyrid-1-yl]-2-méthyl-2-(napht-2-yl)propane et ses sels d'addition d'acides.

8. Procédé pour la préparation d'un composé de formule (I) 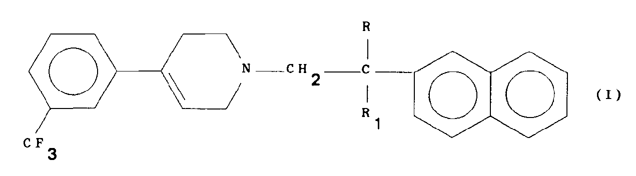 dans laquelle l'un des radicaux R et R₁ est un groupe méthyle et l'autre est un atome d'hydrogène ou un groupe méthyle, et de ses sels d'addition d'acides qui comprend:
a) la réduction de l'amide correspondant de formule (II) 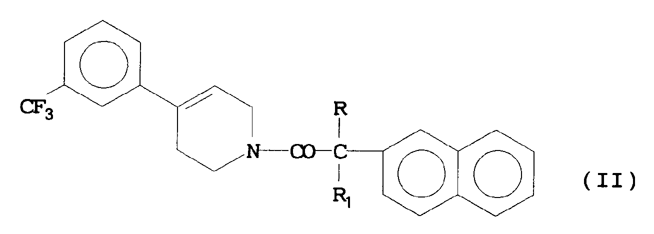 dans laquelle R et R₁ sont tels que définis ci-dessus ; ou
b) la réaction entre la 4-(3-trifluorométhylphényl)-1,2,3,6-tétrahydropyridine de formule (III) 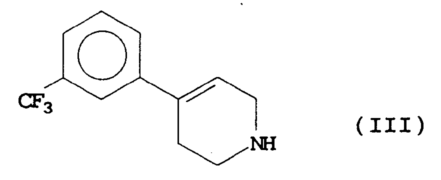 et un composé de formule (V) 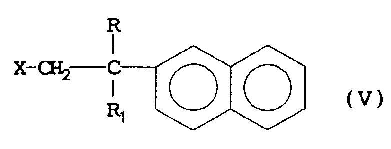 dans laquelle R et R₁ sont tels que définis ci-dessus et X représente un atome de chlore, brome, ou iode ou un groupe attracteur d'électrons ;
ou
c) quand on veut obtenir un composé de formule (I) dans laquelle l'un des radicaux R et R₁ est un groupe méthyle et l'autre est un atome d'hydrogène, la réaction entre le composé de formule (III) et le composé (VI) 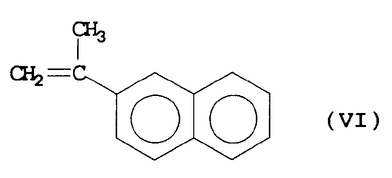 en présence d'un agent de condensation basique ;
éventuellement suivie par la conversion du produit ainsi obtenu par l'un de ses sels d'addition d'acides.

9. Procédé pour la préparation d'une composition pharmaceutique renfermant en tant que principe actif au moins un composé de formule (I) 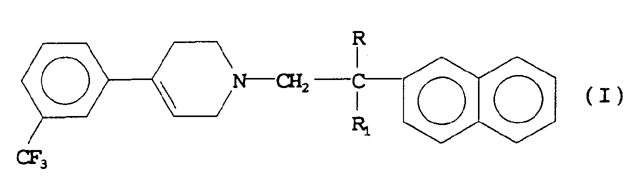 dans laquelle l'un des radicaux R et R₁ est un groupe méthyle et l'autre est un atome d'hydrogène ou un groupe méthyle ou un de ses sels d'addition d'acides pharmaceutiquement acceptables, qui consiste à mélanger ledit principe actif avec un excipient pharmaceutiquement acceptable.

10. Composé de formule (II) 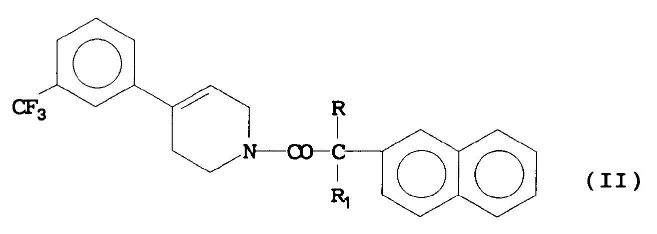 dans laquelle l'un des radicaux R et R₁ est un groupe méthyle et l'autre est un atome d'hydrogène ou un groupe méthyle.

## Patentansprüche (Patentansprüche für folgende(n) Vertragsstaat(en): AT, BE, CH, DE, DK, FR, GB, IT, LI, LU, NL, PT, SE)

1. Verwendung mindestens einer Verbindung der Formel (I) 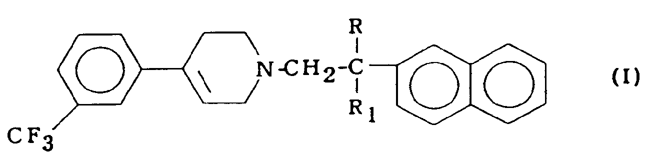 worin R und R₁, unabhängig voneinander, ein Wasserstoffatom oder eine Methylgruppe darstellen, oder eines ihrer pharmazeutisch akzeptablen Säureadditionssalze zur Herstellung eines Medikaments zur Behandlung und/oder Prophylaxe sämtlicher Erkrankungen, bei denen freie Radikale eine Rolle spielen.

2. Verwendung nach Anspruch 1 zur Herstellung eines Medikaments zur Behandlung und/oder Prophylaxe pathologischer Prozesse, die Zellschädigungen bewirken.

3. Verwendung nach Anspruch 2 zur Herstellung eines Medikaments zur Kontrolle und Prophylaxe von zerebraler Degeneration und Degeneration des Rückenmarks nach einer Verletzung, von Schädigungen aufgrund von Ischämie-Reperfusion, von Hornhaut- oder Netzhautschädigungen aufgrund von Sauerstoffradikalen, von Organschädigungen im Zuge von Transplantationen, von durch Strahlung hervorgerufenen Schädigungen, von Rückfällen bei Zwölffingerdarmgeschwüren, von durch freie Radikale bildende toxische Mittel hervorgerufenen Gewebeschädigungen und von durch freie Radikale in entzündlichen Gelenksrheumatoiden bewirkten Schädigungen.

4. Verwendung nach einem der Ansprüche 1 bis 3, worin die Verbindung der Formel (I) 1-[4-(3-Trifluormethylphenyl)-1,2,3,6-tetrahydropyrid-1-yl]-2-(naphth-2-yl)-ethan oder eines seiner pharmazeutisch akzeptablen Säureadditionssalze ist.

5. Verbindung der Formel (I) 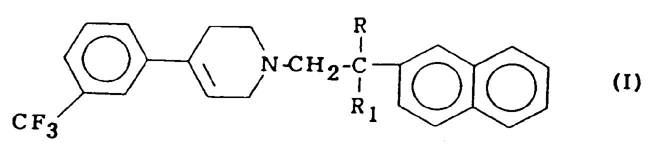 worin einer der Reste R und R₁ eine Methylgruppe und der andere ein Wasserstoffatom oder eine Methylgruppe ist, und ihre Säureadditionssalze.

6. Verbindung nach Anspruch 5, ausgewählt aus 1-[4-(3-Trifluormethylphenyl)-1,2,3,6-tetrahydropyrid-1-yl]-2-(naphth-2-yl)-propan und dessen Säureadditionssalzen.

7. Verbindung nach Anspruch 5, ausgewählt aus 1-[4-(3-Trifluormethylphenyl)-1,2,3,6-tetrahydropyrid-1-yl]-2-methyl-2-(naphth-2-yl)-propan und dessen Säureadditionssalzen.

8. Verfahren zur Herstellung einer Verbindung der Formel (I) 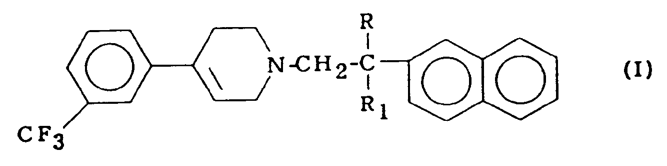 worin einer der Reste R und R₁ eine Methylgruppe und der andere ein Wasserstoffatom oder eine Methylgruppe ist, und ihrer Säureadditionssalze, welches umfaßt:
a) die Reduktion des entsprechenden Amids der Formel (II) 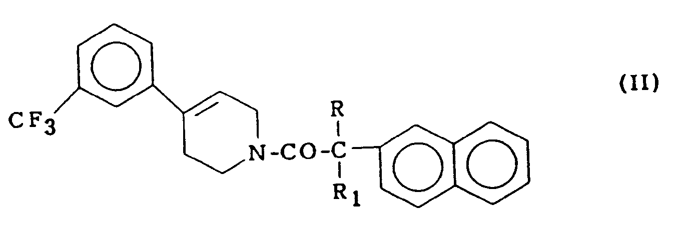 worin R und R₁ wie oben definiert sind; oder
b) die Umsetzung von 4-(3-Trifluormethylphenyl)-1,2,3,6-tetrahydropyridin der Formel (III) 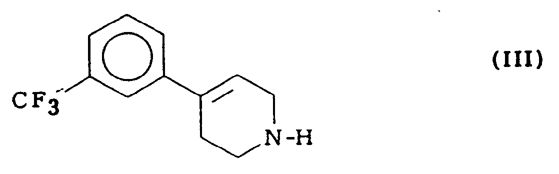 mit einer Verbindung der Formel (V) 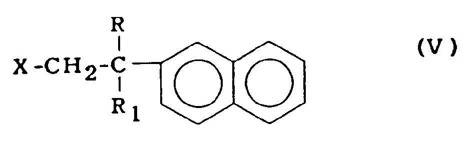 worin R und R₁ wie oben definiert sind und X ein Chlor-, Brom- oder Iodatom oder eine Elektronen anziehende Gruppe darstellt;
oder
c) wenn man eine Verbindung der Formel (I) erhalten will, in der einer der Reste R und R₁ eine Methylgruppe und der andere ein Wasserstoffatom ist, die Umsetzung der Verbindung der Formel (III) mit der Verbindung (VI) 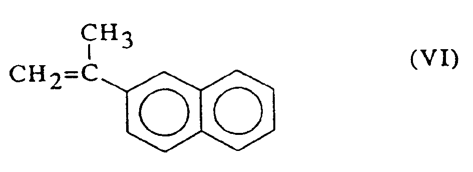
in Gegenwart eines basischen Kondensiermittels;
gegebenenfalls gefolgt von der Überführung des so erhaltenen Produkts in eines seiner Säureadditionssalze.

9. Pharmazeutische Zusammensetzung, die als Wirkstoff mindestens eine Verbindung der Formel (I) 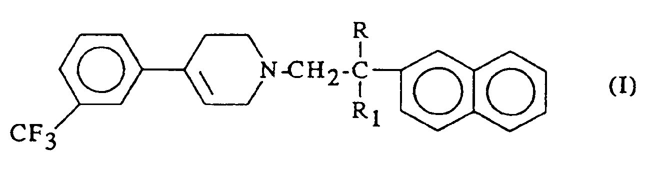 worin einer der Reste R und R₁ eine Methylgruppe und der andere ein Wasserstoffatom oder eine Methylgruppe ist, oder eines ihrer pharmazeutisch akzeptablen Säureadditionssalze enthält.

10. Verbindung der Formel (II) 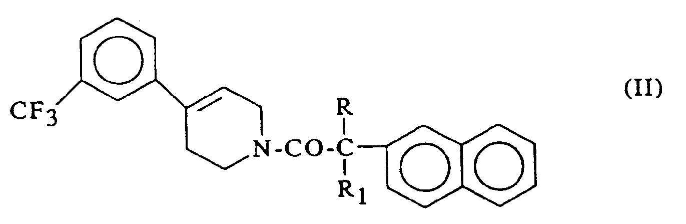 worin einer der Reste R und R₁ eine Methylgruppe und der andere ein Wasserstoffatom oder eine Methylgruppe ist.

## Patentansprüche (Patentansprüche für folgende(n) Vertragsstaat(en): ES)

1. Verwendung mindestens einer Verbindung der Formel (I) 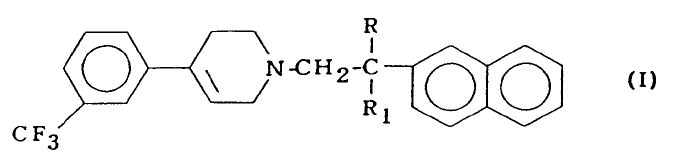 worin R und R₁, unabhängig voneinander, ein Wasserstoffatom oder eine Methylgruppe darstellen, oder eines ihrer pharmazeutisch akzeptablen Säureadditionssalze zur Herstellung eines Medikaments zur Behandlung und/oder Prophylaxe sämtlicher Erkrankungen, bei denen freie Radikale eine Rolle spielen.

2. Verwendung nach Anspruch 1 zur Herstellung eines Medikaments zur Behandlung und/oder Prophylaxe pathologischer Prozesse, die Zellschädigungen bewirken.

3. Verwendung nach Anspruch 2 zur Herstellung eines Medikaments zur Kontrolle und Prophylaxe von zerebraler Degeneration und Degeneration des Rückenmarks nach einer Verletzung, von Schädigungen aufgrund von Ischämie-Reperfusion, von Hornhaut- oder Netzhautschädigungen aufgrund von Sauerstoffradikalen, von Organschädigungen im Zuge von Transplantationen, von durch Strahlung hervorgerufenen Schädigungen, von Rückfällen bei Zwölffingerdarmgeschwüren, von durch freie Radikale bildende toxische Mittel hervorgerufenen Gewebeschädigungen und von durch freie Radikale in entzündlichen Gelenksrheumatoiden bewirkten Schädigungen.

4. Verwendung nach einem der Ansprüche 1 bis 3, worin die Verbindung der Formel (I) 1-[4-(3-Trifluormethylphenyl)-1,2,3,6-tetrahydropyrid-1-yl]-2-(naphth-2-yl)-ethan oder eines seiner pharmazeutisch akzeptablen Säureadditionssalze ist.

5. Verfahren zur Herstellung einer Verbindung der Formel (I) 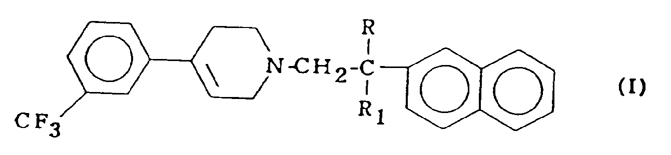 worin einer der Reste R und R₁ eine Methylgruppe und der andere ein Wasserstoffatom oder eine Methylgruppe ist, und ihrer Säureadditionssalze, welches umfaßt:
a) die Reduktion des entsprechenden Amids der Formel (II) 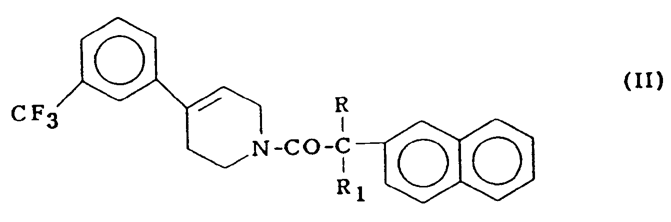 worin R und R₁ wie oben definiert sind; oder
b) die Umsetzung von 4-(3-Trifluormethylphenyl)-1,2,3,6-tetra- hydropyridin der Formel (III) 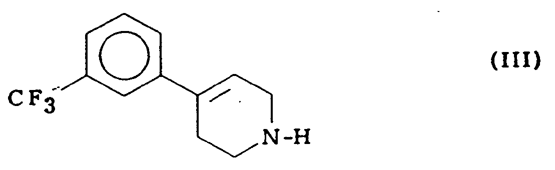 mit einer Verbindung der Formel (V) 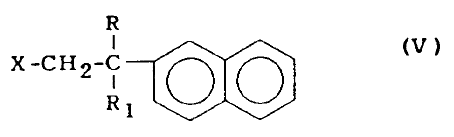 worin R und R₁ wie oben definiert sind und X ein Chlor-, Brom- oder Iodatom oder eine Elektronen anziehende Gruppe darstellt;
oder
c) wenn man eine Verbindung der Formel (I) erhalten will, in der einer der Reste R und R₁ eine Methylgruppe und der andere ein Wasserstoffatom ist, die Umsetzung der Verbindung der Formel (III) mit der Verbindung (VI) 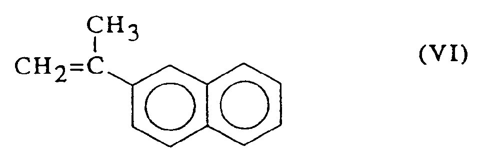
in Gegenwart eines basischen Kondensiermittels;
gegebenenfalls gefolgt von der Überführung des so erhaltenen Produkts in eines seiner Säureadditionssalze.

6. Verfahren nach Anspruch 5 zur Herstellung von 1-[4-(3-Trifluormethylphenyl)-1,2,3,6-tetrahydropyrid-1-yl]-2-(naphth-2-yl)-propan oder einem seiner Säureadditionssalze, welches darin besteht, daß das entsprechende Amid der Formel 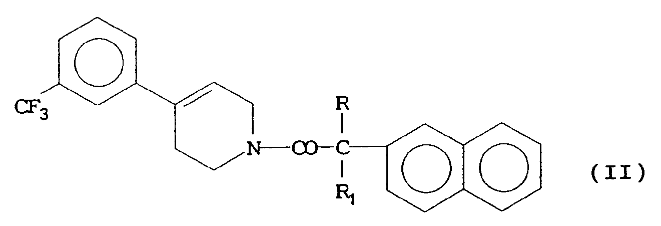 worin R ein Wasserstoffatom und R₁ eine Methylgruppe ist, reduziert wird und gegebenenfalls das so erhaltene Produkt in eines seiner Säureadditionssalze übergeführt wird.

7. Verfahren nach Anspruch 5 zur Herstellung von 1-[4-(3-Trifluormethylphenyl)-1,2,3,6-tetrahydropyrid-1-yl]-2-methyl-2-(naphth-2-yl)-propan oder einem seiner Säureadditionssalze, welches darin besteht, daß das entsprechende Amid der Formel 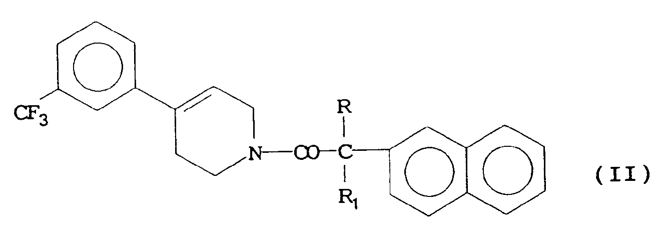 worin R und R₁ jeweils eine Methylgruppe sind, reduziert wird und gegebenenfalls das so erhaltene Produkt in eines seiner Säureadditionssalze übergeführt wird.

8. Verfahren zur Herstellung einer pharmazeutischen Zusammensetzung, die als Wirkstoff mindestens eine Verbindung der Formel (I) 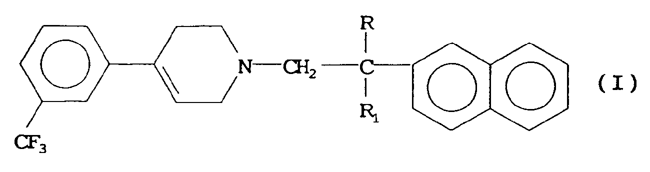 worin einer der Reste R und R₁ eine Methylgruppe und der andere ein Wasserstoffatom oder eine Methylgruppe ist, oder eines ihrer pharmazeutisch akzeptablen Säureadditionssalze enthält, welches darin besteht, daß der Wirkstoff mit einem pharmazeutisch akzeptablen Exzipienten vermischt wird.

9. Verfahren zur Herstellung einer Verbindung der Formel (II) 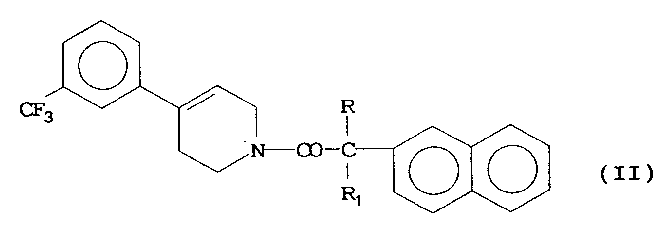 worin einer der Reste R und R₁ eine Methylgruppe und der andere ein Wasserstoffatom oder eine Methylgruppe ist,
welches darin besteht, daß 4-(3-Trifluormethylphenyl)-1,2,3,6-tetrahydropyridin der Formel (III) 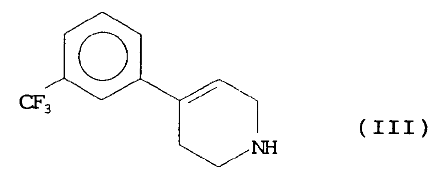 mit einem funktionellen Carbonsäurederivat der Formel (IV) 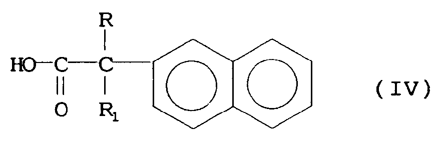 worin R und R₁ wie oben definiert sind, in einem organischen Lösungsmittel bei einer Temperatur zwischen -10°C und der Rückflußtemperatur der Reaktionsmischung reagieren gelassen wird.

## Patentansprüche (Patentansprüche für folgende(n) Vertragsstaat(en): GR)

1. Verwendung mindestens einer Verbindung der Formel (I) 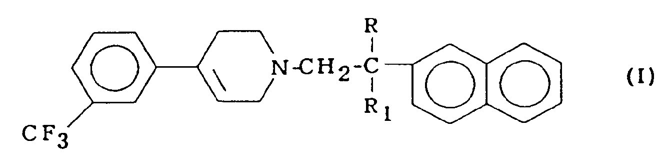 worin R und R₁, unabhängig voneinander, ein Wasserstoffatom oder eine Methylgruppe darstellen, oder eines ihrer pharmazeutisch akzeptablen Säureadditionssalze zur Herstellung eines Medikaments zur Behandlung und/oder Prophylaxe sämtlicher Erkrankungen, bei denen freie Radikale eine Rolle spielen.

2. Verwendung nach Anspruch 1 zur Herstellung eines Medikaments zur Behandlung und/oder Prophylaxe pathologischer Prozesse, die Zellschädigungen bewirken.

3. Verwendung nach Anspruch 2 zur Herstellung eines Medikaments zur Kontrolle und Prophylaxe von zerebraler Degeneration und Degeneration des Rückenmarks nach einer Verletzung, von Schädigungen aufgrund von Ischämie-Reperfusion, von Hornhaut- oder Netzhautschädigungen aufgrund von Sauerstoffradikalen, von Organschädigungen im Zuge von Transplantationen, von durch Strahlung hervorgerufenen Schädigungen, von Rückfällen bei Zwölffingerdarmgeschwüren, von durch freie Radikale bildende toxische Mittel hervorgerufenen Gewebeschädigungen und von durch freie Radikale in entzündlichen Gelenksrheumatoiden bewirkten Schädigungen.

4. Verwendung nach einem der Ansprüche 1 bis 3, worin die Verbindung der Formel (I) 1-[4-(3-Trifluormethylphenyl)-1,2,3,6-tetrahydropyrid-1-yl]-2-(naphth-2-yl)-ethan oder eines seiner pharmazeutisch akzeptablen Säureadditionssalze ist.

5. Verbindung der Formel (I) 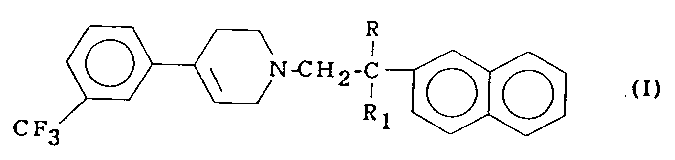 worin einer der Reste R und R₁ eine Methylgruppe und der andere ein Wasserstoffatom oder eine Methylgruppe ist, und ihre Säureadditionssalze.

6. Verbindung nach Anspruch 5, ausgewählt aus 1-[4-(3-Trifluormethylphenyl)-1,2,3,6-tetrahydropyrid-1-yl]-2-(naphth-2-yl)-propan und dessen Säureadditionssalzen.

7. Verbindung nach Anspruch 5, ausgewählt aus 1-[4-(3-Trifluormethylphenyl)-1,2,3,6-tetrahydropyrid-1-yl]-2-methyl-2-(naphth-2-yl)-propan und dessen Säureadditionssalzen.

8. Verfahren zur Herstellung einer Verbindung der Formel (I) 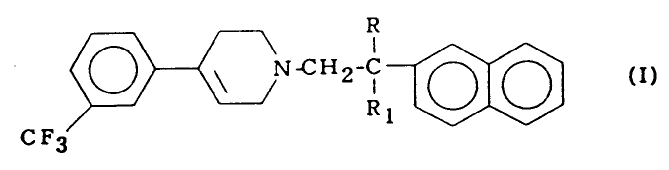 worin einer der Reste R und R₁ eine Methylgruppe und der andere ein Wasserstoffatom oder eine Methylgruppe ist, und ihrer Säureadditionssalze, welches umfaßt:
a) die Reduktion des entsprechenden Amids der Formel (II) 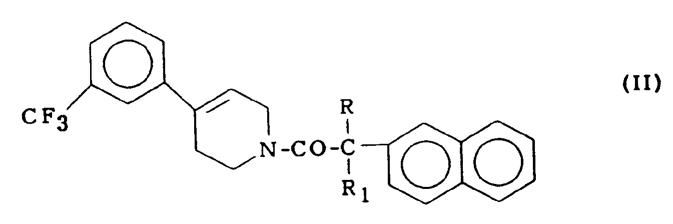 worin R und R₁ wie oben definiert sind; oder
b) die Umsetzung von 4-(3-Trifluormethylphenyl)-1,2,3,6-tetrahydropyridin der Formel (III) 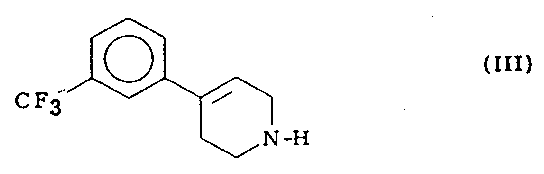 mit einer Verbindung der Formel (V) 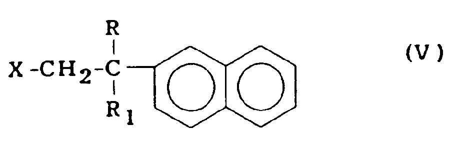 worin R und R₁ wie oben definiert sind und X ein Chlor-, Brom- oder Iodatom oder eine Elektronen anziehende Gruppe darstellt;
oder
c) wenn man eine Verbindung der Formel (I) erhalten will, in der einer der Reste R und R₁ eine Methylgruppe und der andere ein Wasserstoffatom ist, die Umsetzung der Verbindung der Formel (III) mit der Verbindung (VI) 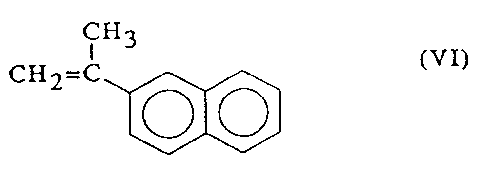
in Gegenwart eines basischen Kondensiermittels;
gegebenenfalls gefolgt von der Überführung des so erhaltenen Produkts in eines seiner Säureadditionssalze.

9. Verfahren zur Herstellung einer pharmazeutischen Zusammensetzung, die als Wirkstoff mindestens eine Verbindung der Formel (I) 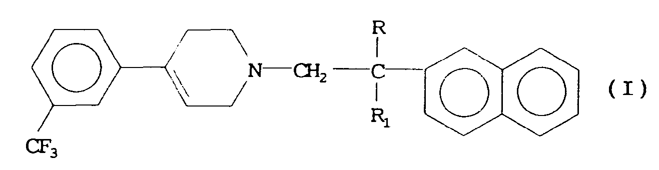 worin einer der Reste R und R₁ eine Methylgruppe und der andere ein Wasserstoffatom oder eine Methylgruppe ist, oder eines ihrer pharmazeutisch akzeptablen Säureadditionssalze enthält, welches darin besteht, daß der Wirkstoff mit einem pharmazeutisch akzeptablen Exzipienten vermischt wird.

10. Verbindung der Formel (II) 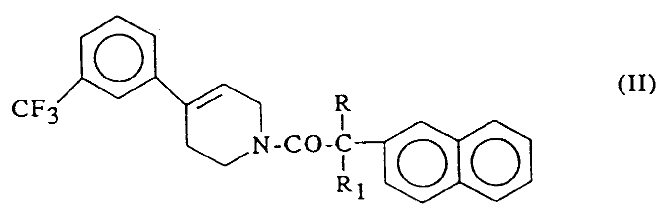 worin einer der Reste R und R₁ eine Methylgruppe und der andere ein Wasserstoffatom oder eine Methylgruppe ist.

## Claims (Claims for the following Contracting State(s): AT, BE, CH, DE, DK, FR, GB, IT, LI, LU, NL, PT, SE)

1. Use of at least one compound of formula (I) 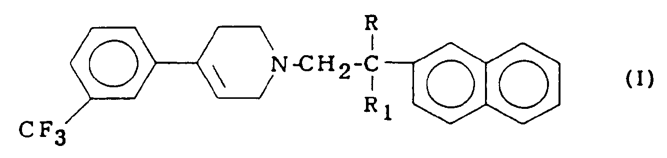 in which R and R₁, each independently, represent a hydrogen atom or a methyl group, or of a pharmaceutically acceptable acid addition salt thereof for the preparation of a medicament for the treatment and/or prevention of any pathological conditions implicating free radicals.

2. Use according to claim 1 for the preparation of a medicament for the treatment and/or prevention of pathological processes involving cell damage.

3. Use according to claim 2 for the preparation of a medicament for the control and/or prevention of post-injury degeneration of the brain and spinal cord, ischemic-reperfusion injury, damage in the comea or retina due to oxygen radicals, organ damage in transplantations, cell damage by radiation, relapse of duodenal ulcers, tissue damage due to toxic agents which produce free radicals, and injury by free radicals in inflamed rheumatoid joints.

4. Use according to any one of claims 1 to 3, in which the compound of formula (I) is 1-[4-(3-trifluoromethylphenyl)-1,2,3,6-tetrahydropyrid-1-yl]-2-(naphth-2-yl)ethane or a pharmaceutically acceptable acid addition salt thereof.

5. Compound of formula (I) 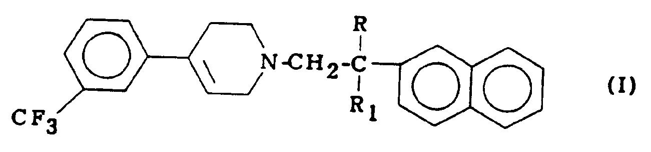 in which one of the radicals R and R₁ is a methyl group and the other is a hydrogen atom or a methyl group, and its acid addition salts.

6. Compound according to claim 5 selected from 1-[4-(3-trifluoromethylphenyl)-1,2,3,6-tetrahydropyrid-1-yl]-2-(naphth-2-yl)propane and its acid addition salts.

7. Compound according to claim 5 selected from 1-[4-(3-trifluoromethylphenyl)-1,2,3,6-tetrahydropyrid-1-yl]-2-methyl-2-(naphth-2-yl)propane and its acid addition salts.

8. Process for the preparation of a compound of formula (I) 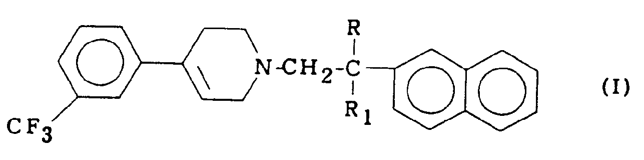 in which one of the radicals R and R₁ is a methyl group and the other is a hydrogen atom or a methyl group and its acid addition salts, which comprises
a) reducing the corresponding amide of formula (II) 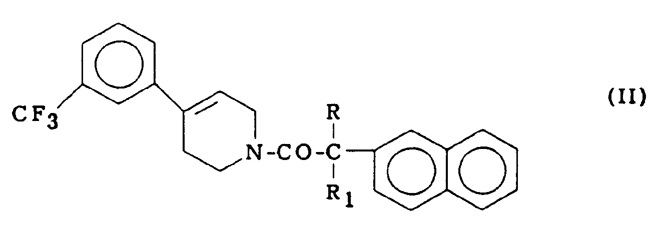 in which R and R₁ are as defined above; or
b) reacting 4-(3-trifluoromethylphenyl)-1,2,3,6-tetrahydropyridine of formula (III) 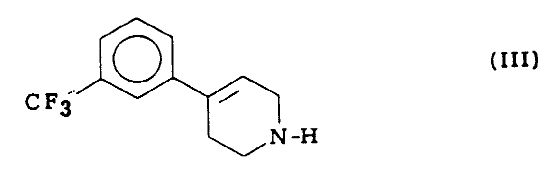 with a compound of formula (V) 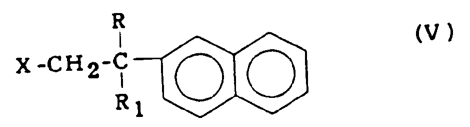 in which R and R₁ are as defined above and X represents a chlorine, bromine, iodine atom or an electron-attracting group;
or
c) when a compound of formula (I) is desired in which one of the radicals R and R₁ is a methyl group and the other is a hydrogen atom, reacting the compound of formula (III) with the compound (VI) 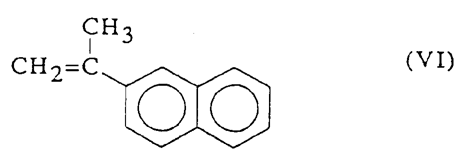 in the presence of a basic condensation agent;
optionally followed by conversion of the thus obtained product into an acid addition salt thereof.

9. Pharmaceutical composition containing as the active principle at least one compound of formula (I) 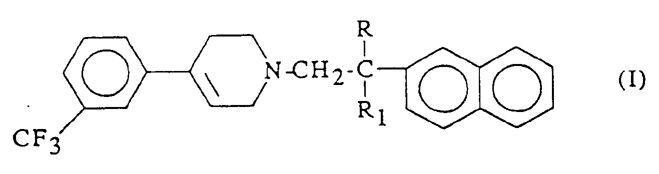 in which one of the radicals R and R₁ is a methyl group and the other is a hydrogen atom or a methyl group, or a pharmaceutically acceptable acid addition salt thereof.

10. Compound of formula (II) 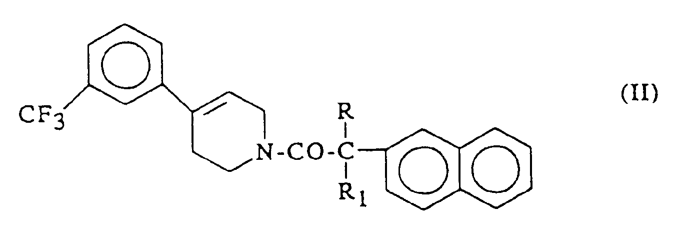 in which one of the radicals R and R₁ is a methyl group and the other is a hydrogen atom or a methyl group.

## Claims (Claims for the following Contracting State(s): ES)

1. Use of at least one compound of formula (I) 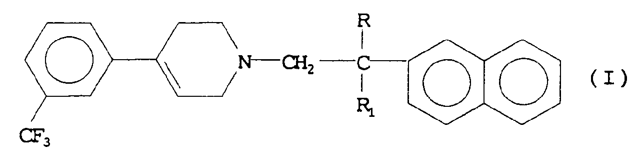 in which R and R₁, each independently, represent a hydrogen atom or a methyl group, or of a pharmaceutically acceptable acid addition salt thereof for the preparation of a medicament for the treatment and/or prevention of any pathological conditions implicating free radicals.

2. Use according to claim 1 for the preparation of a medicament for the treatment and/or prevention of pathological processes involving cell damage.

3. Use according to claim 2 for the preparation of a medicament for the control and/or prevention of post-injury degeneration of the brain and spinal cord, ischemic-reperfusion injury, damage in the comea or retina due to oxygen radicals, organ damage in transplantations, cell damage by radiation, relapse of duodenal ulcers, tissue damage due to toxic agents which produce free radicals, and injury by free radicals in inflamed rheumatoid joints.

4. Use according to any one of claims 1 to 3, in which the compound of formula (I) is 1-[4-(3-trifluoromethylphenyl)-1,2,3,6-tetrahydropyrid-1-yl]-2-(naphth-2-yl)ethane or a pharmaceutically acceptable acid addition salt thereof.

5. Process for the preparation of a compound of formula (I) 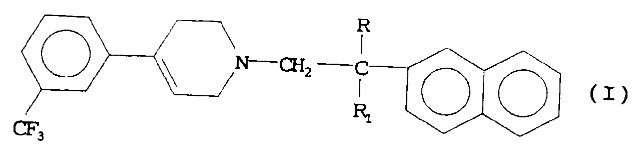 in which one of the radicals R and R₁ is a methyl group and the other is a hydrogen atom or a methyl group and its acid addition salts, which comprises
a) reducing the corresponding amide of formula (II) 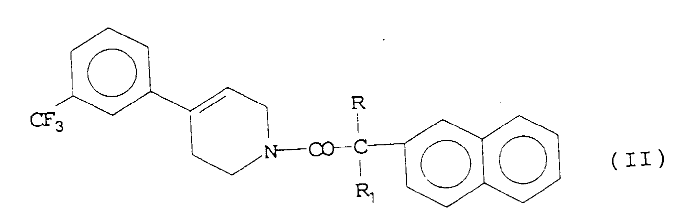 in which R and R₁ are as defined above; or
b) reacting 4-(3-trifluoromethylphenyl)-1,2,3,6-tetrahydropyridine of formula (III) 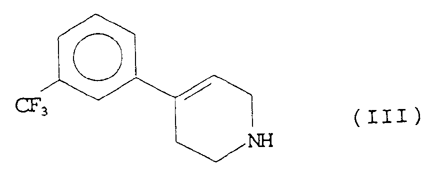 with a compound of formula (V) 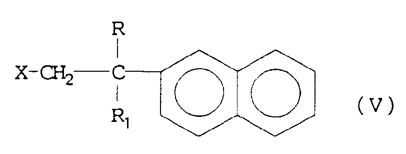 in which R and R₁ are as defined above and X represents a chlorine, bromine, iodine atom or an electron-attracting group;
or
c) when a compound of formula (I) is desired in which one of the radicals R and R₁ is a methyl group and the other is a hydrogen atom, reacting the compound of formula (III) with the compound (VI) 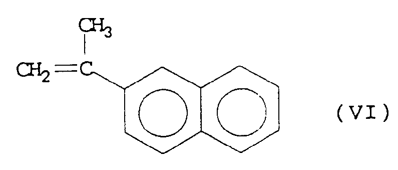 in the presence of a basic condensation agent;
optionally followed by conversion of the thus obtained product into an acid addition salt thereof.

6. Process according to claim 5, for the preparation of 1-[4-(3-trifluoromethylphenyl)-1,2,3,6-tetrahydropyrid-1-yl]-2-(naphth-2-yl)propane or one of its acid addition salts which consists in reducing the corresponding amide of formula: 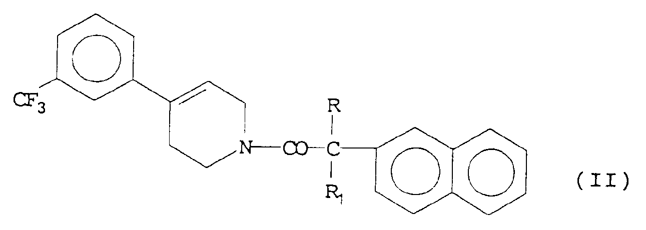 in which R is a hydrogen atom and R₁ is a methyl group,
and in optionally converting the thus obtained product into one of its acid addition salts.

7. Process according to claim 5, for the preparation of 1-[4-(3-trifluoromethylphenyl)-1,2,3,6-tetrahydropyrid-1-yl]-2-methyl-2-(naphth-2-yl)propane or one of its acid addition salts which consists in reducing the corresponding ami de of formula: 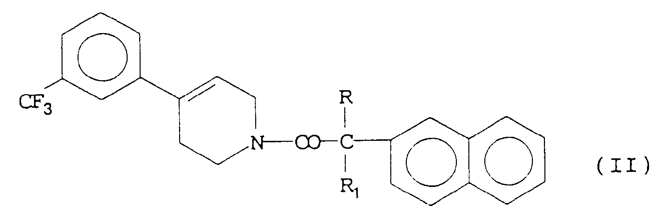 in which R and R₁ are each a methyl group,
and in optionally converting the thus obtained product into one of its acid addition salts.

8. Process for the preparation of a pharmaceutical composition containing as the active principle at least one compound of formula (I) 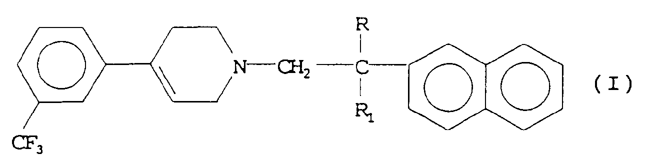 in which one of the radicals R and R₁ is a methyl group and the other is a hydrogen atom or a methyl group or one of its pharmaceutically acceptable acid addition salts, which consists in mixing said active principle with a pharmaceutically acceptable excipient.

9. Process for the preparation of a compound of formula (II) 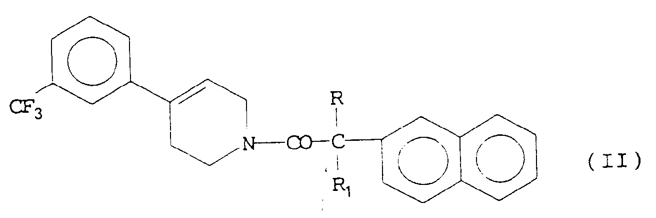 in which one of the radicals R and R₁ is a methyl group and the other is a hydrogen atom or a methyl group,
which consists in reacting the 4-(3-trifluoromethylphenyl)-1,2,3,6-tetrahydropyridine of formula (III) 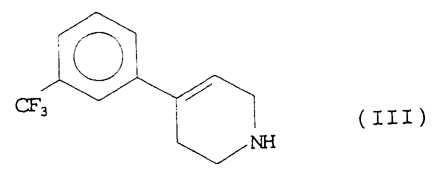 with a functional derivative of the carboxylic acid of formula (IV) 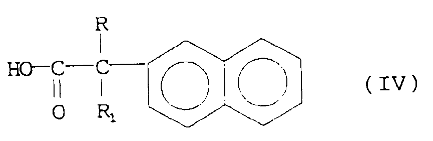 in which R and R₁ are as defined above, in an organic solvent at a temperature ranging between -10°C and the reflux temperature of the reaction mixture.

## Claims (Claims for the following Contracting State(s): GR)

1. Use of at least one compound of formula (I) 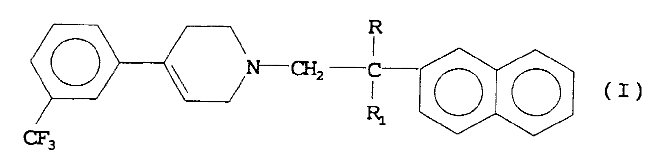 in which R and R₁, each independently, represent a hydrogen atom or a methyl group, or of a pharmaceutically acceptable acid addition salt thereof for the preparation of a medicament for the treatment and/or prevention of any pathological conditions implicating free radicals.

2. Use according to claim 1 for the preparation of a medicament for the treatment and/or prevention of pathological processes involving cell damage.

3. Use according to claim 2 for the preparation of a medicament for the control and/or prevention of post-injury degeneration of the brain and spinal cord, ischemic-reperfusion injury, damage in the comea or retina due to oxygen radicals, organ damage in transplantations, cell damage by radiation, relapse of duodenal ulcers, tissue damage due to toxic agents which produce free radicals, and injury by free radicals in inflamed rheumatoid joints.

4. Use according to any one of claims 1 to 3, in which the compound of formula (I) is 1-[4-(3-trifluoromethylphenyl)-1,2,3,6-tetrahydropyrid-1-yl]-2-(naphth-2-yl)ethane or a pharmaceutically acceptable acid addition salt thereof.

5. Compound of formula (I) 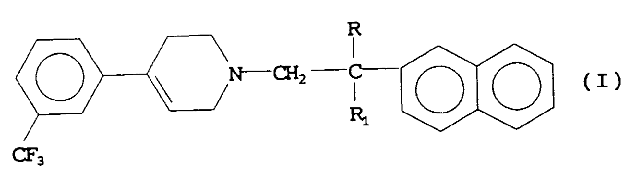 in which one of the radicals R and R₁ is a methyl group and the other is a hydrogen atom or a methyl group, and its acid addition salts.

6. Compound according to claim 5 selected from 1-[4-(3-trifluoromethylphenyl)-1,2,3,6-tetrahydropyrid-1-yl]-2-(naphth-2-yl)propane and its acid addition salts.

7. Compound according to claim 5 selected from 1-[4-(3-trifluoromethylphenyl)-1,2,3,6-tetrahydropyrid-1-yl]-2-methyl-2-(naphth-2-yl)propane and its acid addition salts.

8. Process for the preparation of a compound of formula (I) 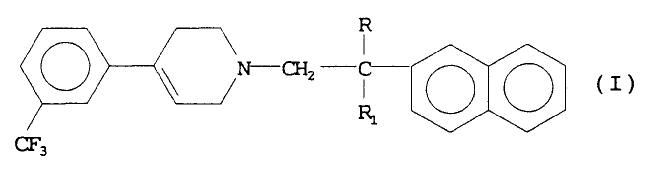 in which one of the radicals R and R₁ is a methyl group and the other is a hydrogen atom or a methyl group and its acid addition salts, which comprises
a) reducing the corresponding amide of formula (II) 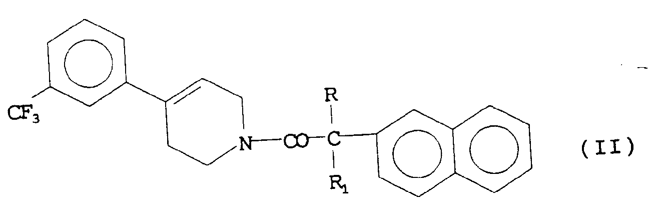 in which R and R₁ are as defined above; or
b) reacting 4-(3-trifluoromethylphenyl)-1,2,3,6-tetrahydropyridine of formula (III) 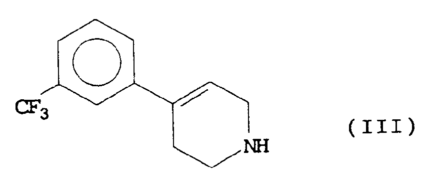 with a compound of formula (V) 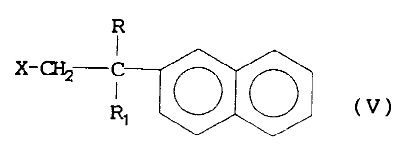 in which R and R₁ are as defined above and X represents a chlorine, bromine, iodine atom or an electron-attracting group;
or
c) when a compound of formula (I) is desired in which one of the radicals R and R₁ is a methyl group and the other is a hydrogen atom, reacting the compound of formula (III) with the compound (VI) 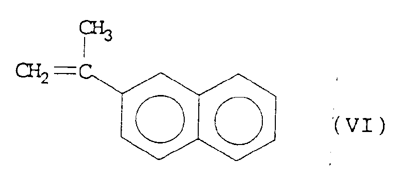 in the presence of a basic condensation agent;
optionally followed by conversion of the thus obtained product into an acid addition salt thereof.

9. Process for the preparation of a pharmaceutical composition containing as the active principle at least one compound of formula (I) 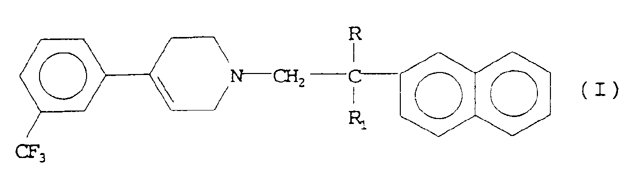 in which one of the radicals R and R₁ is a methyl group and the other is a hydrogen atom or a methyl group, or a pharmaceutically acceptable acid addition salt thereof, which consists in mixing said active principle with a pharmaceutically acceptable excipient.

10. Compound of formula (II) 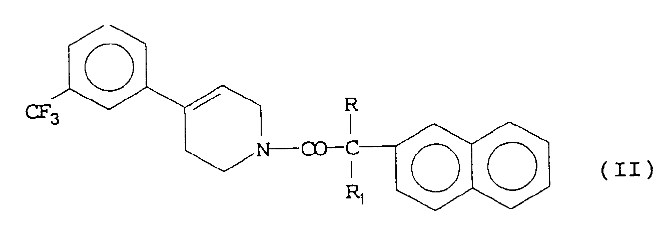 in which one of the radicals R and R₁ is a methyl group and the other is a hydrogen atom or a methyl group.
